# EUROPEAN PATENT APPLICATION

(11) **EP 4 725 398 A1**
(43) Date of publication of application: **15.04.2026**
(21) Application number: 25207760.7
(22) Date of filing: 09.10.2025
(51) Int. Cl.: A61B 5/00, G06F 3/01, G16H 10/60, G16H 40/60

(54) **SYSTEM AND METHOD FOR AUGMENTED REALITY-ENABLED DYNAMIC**

(30) Priority: 10.10.2024 US 202418912429
(71) Applicant: Mitel Networks Corporation, Ottawa, Ontario K2K 3K1 (CA)
(72) Inventor: Jayachandran, Radhakrishnan, 560035 Bengaluru (IN); Murthy, Tejas, 56008 Bangalore (IN); Naidoo, Logendra, Ottawa, K1S 0W8 (CA)
(74) Representative: McDougall, James

(57) **Abstract**

An augmented reality (AR) system is disclosed that can include an AR server configured to create an immersive environment superimposed on the physical environment; a patient device configured to monitor a condition data of a patient, wherein the patient device is in communication with the AR server and is configured to transmit the condition data to the AR server; and an AR object processing engine in communication with the AR server. The AR object processing engine can generate and place an AR object in the immersive environment. The AR object can be selected based on a type of the condition data and is configured to display the condition data. One or more user devices in communication with the AR server, wherein at least one of the one or more user devices is configured to view or display the immersive environment, including the AR object, superimposed on the physical environment.

## Description

### Background

Challenges exist to communicate and collaborate in patient care settings. Traditional methods, such as clipboard and whiteboard notes, can result in delays and inefficiencies in accessing and sharing critical health or medical-related information. This lack of real-time accessibility can hinder patient care and effective teamwork, as vital information is often scattered and difficult to consolidate.

Moreover, healthcare professionals frequently rely on shift-to-shift handovers, which can result in fragmented communication and potential loss of crucial details. While digital communication tools like modem videoconferencing can be used, they are often incompatible with the dynamic and immediate needs of a healthcare environment. These tools also fail to provide a continuous, seamless stream of communication, making it challenging to maintain a cohesive understanding of patient care and treatment plans.

The absence of an integrated, real-time communication system exacerbates these issues, leading to inefficiencies and potential errors in patient care since managing the paper trail is tedious. The need for a solution that bridges the gap between traditional notetaking methods and modern digital communication tools is evident.

The solution of this disclosure resolves these and other issues of the art.

### Summary

A first aspect of this disclosure provides an augmented reality (AR) system configured to virtually position a virtual object into a physical environment, comprising: an AR server configured to create an immersive environment superimposed on the physical environment; a patient device configured to monitor a condition data of a patient, wherein the patient device is in communication with the AR server and is configured to transmit the condition data to the AR server; an AR object processing engine in communication with the AR server, wherein the AR object processing engine is configured to generate and place an AR object in the immersive environment, wherein the AR object is selected based on a type of the condition data and is configured to display the condition data; and one or more user devices in communication with the AR server, wherein at least one of the one or more user devices is configured to view or display the immersive environment, including the AR object, superimposed on the physical environment.

The AR system may further comprise: a plurality of patient devices and the AR object processing engine may be configured to generate and virtually position a plurality of AR objects into the physical environment, wherein each of the plurality of AR objects is based on a separate type of condition than the other of the plurality of AR objects and each of the plurality of AR objects is configured to display condition data for the separate type of condition to which it relates.

The patient device may be a wearable device positioned on the patient or on an article of clothing of the patient.

At least one of the one or more user devices may be further configured to change the AR object to another AR object, wherein the another AR object may be configured to display a different condition data from that displayed by the AR object.

Either (a) the one or more user devices are, or (b) the AR server may be further configured to combine respective condition data from the plurality of the AR objects in order to create a combined condition data indicative of a medical event.

The AR object processing engine may be further configured to generate and place a combined AR object into the immersive environment and the combined AR object may be configured to display the combined condition data.

The AR system may further comprise: a plurality of users, wherein each of the plurality of users is associated with a unique one of the one or more user devices, and each of the plurality of users has a permission rating identified by dynamic permission management logic that regulates access to the AR object and regulates an ability to change the AR object.

The system may further comprise: an AR database in communication with the AR object, wherein the AR database saves contact information associated with one or more users who have (a) interacted with the AR object, or (b) are affiliated with an aspect of the displayed condition data; and a telephony server in communication with the AR database and the one or more user devices; wherein the one or more user devices are configured to access the AR database and using the telephony server initiate a communication with at least one of the one or more users, wherein the communication comprises placing a phone call, initiating a conference call, starting a chat session, or scheduling a future meeting.

At least one of the one or more user devices may be remote to the physical environment.

The AR system may further comprise: a plurality of user devices and each of the plurality of user devices may be configured to communicate with each of the other of the plurality of user devices.

A second aspect of this disclosure provides a computerized method utilizing an augmented reality (AR) system to virtually position an AR object into a physical environment, wherein the method comprises the steps of: a scanner assessing the physical environment to identify one or more suitable locations for virtually positioning the AR object into the physical environment, wherein the scanner communicates the one or more suitable locations to one or both of an AR server and an AR object processing engine; a patient device monitoring a condition of a patient, wherein the patient device is positioned on the patient or on clothing of the patient, and the patient device communicates patient condition data to one or both of the AR server and the AR object processing engine; developing, by the AR object processing engine, an AR object customized to display the patient condition data; generating, by the AR server, an immersive environment superimposed on the physical environment; virtually positioning the AR object at one of the one or more suitable locations; and displaying the condition data on the AR object.

The AR object processing engine may generate a plurality of the AR objects and position them as breadcrumbing focal points using a persistent marking mechanism distributed at contextual locations in the physical environment, wherein as a user device moves through networked areas of the physical environment, the AR object becomes accessible to the user device for communication and collaboration, enabling continuous real-time collaboration, only if a permission relationship is determined based on attributes of the AR object, interactions by the user device, and a permission rating of a user and/or the user device.

The scanner may identify one or more suitable locations for each of a plurality of different AR objects.

Each of a plurality of users may be assigned a unique user device, wherein each unique user device is configured to view or display the immersive environment and the AR object.

The method may further comprise implementing permissions for each user of the immersive environment in order to regulate access to the AR object.

Each user device may further enable, based on the permission assigned to each unique user, the unique user to interact with the AR object.

A third aspect of this disclosure provides an augmented reality (AR) apparatus that comprises (a) an AR server, (b) a patient device configured to monitor a condition data of a patient, (c) an object processing engine in communication with the AR server, and (d) a processor and a tangible, non-transitory memory configured to communicate with the processor, the non-transitory memory having stored thereon instructions which, when executed by the processor, are configured to cause the AR apparatus to execute a method including the following steps: the AR server generating an immersive environment superimposed on a physical environment; the patient device monitoring and transmitting condition data to one or both of the AR server and the object processing engine; the object processing engine creating and virtually positioning an AR object in the physical environment, wherein creating the AR object is based on a type of the condition data; and displaying the condition data on the AR object.

The AR object may comprise controls to vary a format or a view of the condition data, or to instruct the AR object to display a different condition data.

The instructions, when executed by the processor, may be further configured to permit access by users having a user device to the immersive environment and the AR object, wherein each user device displays, or enables a user to view, the immersive environment and the AR object.

The instructions, when executed by the processor, may be further configured to limit access to the AR object to users using dynamic permission management logic so that that sensitive information related to or from the AR object is accessible only to users with user permissions identified based on user roles and/or attributes of the AR object.

### Brief Description of the Drawings

The subject matter of the present disclosure is particularly pointed out and distinctly claimed in the concluding portion of the specification. A more complete understanding of the present disclosure, however, may best be obtained by referring to the detailed description and claims when considered in connection with the drawing figures, wherein like numerals denote like elements and wherein:
FIG. 1 is a block diagram of a system according to aspects of this disclosure.
FIG. 2 is a block diagram of a method for utilizing an AR system to virtually position an AR object into a physical environment.
FIG. 3 is a method for utilizing an AR system.

It will be appreciated that elements in the figures are illustrated for simplicity and clarity and have not necessarily been drawn to scale. For example, the dimensions of some of the elements in the figures may be exaggerated relative to other elements to help to improve understanding of illustrated embodiments of the present invention.

### Detailed Description

The description of exemplary embodiments of the present invention provided herein is merely exemplary and is intended for purposes of illustration only; the following description is not intended to limit the scope of the invention as claimed. Moreover, recitation of multiple embodiments having stated features is not intended to exclude other embodiments having additional features or other embodiments incorporating different combinations of the stated features.

It must also be noted that, the term "exemplary" is used in the sense of "example," rather than "ideal."

It must also be noted that, as used in the specification and the appended claims, the singular forms "a," "an" and "the" include plural referents unless the context clearly dictates otherwise.

By "comprising" or "containing" or "including" it is meant that at least the named compound, element, particle, or method step is present in the composition or article or method, but does not exclude the presence of other compounds, materials, particles, method steps, even if the other such compounds, material, particles, method steps have the same function as what is named.

Relative terms, such as "about," "substantially," or "approximately" are used to include small variations with specific numerical values (e.g., +/- x%,), as well as including the situation of no variation (+/-0%). In various embodiments, the numerical value x is less than or equal to 10 - e.g., less than or equal to 5, to 2, to 1, or smaller.

As used herein, "database" refers to any suitable database for storing information, electronic files or code to be utilized to practice embodiments of this disclosure. As used herein, "server" refers to any suitable server, computer or computing device for performing functions utilized to practice embodiments of this disclosure.

As used herein, "software" refers to programs or other operating information utilized by a processor or other computing hardware.

As used herein, the term "Augmented Reality" or AR is defined as an interactive experience of a real-world environment where the user is provided with additional computer-generated information that enhances their perception of reality.

As used herein the term "Virtual Reality" is defined as a simulated experience that can be similar to or completely different from the real world and the surrounding environment is completely virtual.

This disclosure provides a system that integrates advanced communication with a collaboration system utilizing AR technology to integrate virtual objects into physical spaces. This system enables users (e.g., users and other stakeholders in the healthcare sector) to access and interact with individual-specific data (e.g., patient data) and other relevant information in real-time, using wearables, including AR-enabled devices. In some aspects, by placing virtual objects within the physical environment, users (e.g., healthcare professionals) can access and update individual information (e.g., patient information), individual plans (e.g., treatment plans), and procedural guidelines directly at the point of care. In some aspects, the systems and methods of this disclosure can be configured to support continuous collaboration and allow multiple users to interact with the same virtual objects over time. This approach not only streamlines patient care but also fosters interdisciplinary teamwork, improving overall efficiency and patient outcomes. While examples herein are described with respect to healthcare environments, the systems and methods are not so limited and can be applied to any environment where collaboration is essential.

The disclosure can include an AR system configured to virtually position a virtual object into a physical environment, wherein the AR system can include an AR server configured to create an immersive environment superimposed on the physical environment. A patient device can monitor a condition data of a patient, wherein the patient device can be in communication with the AR server and transmit the condition data to the AR server. An AR object processing engine can be in communication with the AR server, wherein the AR object processing engine can generate and place an AR object in the immersive environment. The AR object is selected based on a type of the condition data and is configured to display the condition data. One or more user devices can be in communication with the AR server. At least one of the one or more user devices can view or display the immersive environment, including the AR object, superimposed on the physical environment.

The disclosure can also include a computerized method utilizing an AR system to virtually position an AR object into a physical environment. The computerized method can include a scanner assessing the physical environment to identify one or more suitable locations for virtually positioning the AR object into the physical environment, wherein the scanner communicates the one or more suitable locations to one or both of an AR server and an AR object processing engine; a patient device monitoring a condition of a patient, wherein the patient device is positioned on the patient or on clothing of the patient, and the patient device communicates patient condition data to one or both of the AR server and the AR object processing engine; developing, by the AR object processing engine, an AR object customized to display the patient condition data; generating, by the AR server, an immersive environment superimposed on the physical environment; virtually positioning the AR object at one of the one or more suitable locations; and displaying the condition data on the AR object.

The disclosure can also include an AR apparatus that includes (a) an AR server, (b) a patient device configured to monitor a condition data of a patient, (c) an object processing engine in communication with the AR server, and (d) a processor and a tangible, non-transitory memory configured to communicate with the processor. The non-transitory memory can include stored thereon instructions which, when executed by the processor, are configured to cause the AR apparatus to execute a method including the AR server generating an immersive environment superimposed on a physical environment; the patient device monitoring and transmitting condition data to one or both of the AR server and the object processing engine; the object processing engine creating and virtually positioning an AR object in the physical environment, wherein creating the AR object is based on a type of the condition data; and displaying the condition data on the AR object.

As used herein, "tangible, non-transitory memory" refers to computer program instructions, encoded on computer storage medium for execution by, or to control the operation of, data processing apparatus. Alternatively, or additionally, the program instructions can be encoded on an artificially-generated propagated signal, e.g., a machine-generated electrical, optical, or electromagnetic signal, which is generated to encode information for transmission to a suitable receiver apparatus for execution by a data processing apparatus. A computer storage medium can be, or be included in, a computer-readable storage device, a computer-readable storage substrate, a random or serial access memory array or device, or a combination of one or more of the substrates and devices. By way of example and not limitation, computer storage media (also referred to herein as "computer-readable storage medium" or "computer-readable storage media") may include volatile and non-volatile, removable and non-removable media implemented in any method or technology for storage of information such as computer-storage instructions, data structures, program modules, or other data.

Moreover, while a computer storage medium is not a propagated signal, a computer storage medium can be a source or destination of computer program instructions encoded in an artificially-generated propagated signal. The computer storage medium can also be, or be included in, one or more separate physical components or media (e.g., solid-state memory that forms part of a device, disks, or other storage devices). In accordance with examples of the disclosure, a non-transient computer readable medium containing program can perform functions of one or more methods, modules, engines and/or other system components as described herein. The computer storage medium can also be, or be included in, random access memory (RAM), read-only memory (ROM), electronically erasable programmable ROM (EEPROM), flash memory or other memory technology, compact disc ROM (CD-ROM), digital versatile disks (DVD) or other optical storage, magnetic cassettes, magnetic tape, magnetic disk storage or other magnetic storage devices, or any other tangible, physical medium which can be used to store computer readable information. Computer storage media", "computer-readable storage medium" or "computer-readable storage media" as described herein do not include transitory signals.

As used herein, the terms "component," "engine," "model," "module," "system," "application", "server," "processor," "memory," and the like are intended to include one or more computer-related units, such as but not limited to hardware, firmware, a combination of hardware and software, software, or software in execution. They can also or in addition refer to computer program instructions, encoded on computer storage medium for execution by, or to control the operation of, data processing apparatus. Alternatively, or additionally, the program instructions can be encoded on an artificially-generated propagated signal, e.g., a machine-generated electrical, optical, or electromagnetic signal, which is generated to encode information for transmission to suitable receiver apparatus for execution by a data processing apparatus. A computer storage medium can be, or be included in, a computer-readable storage device, a computer-readable storage substrate, a random or serial access memory array or device, or a combination of one or more of the substrates and devices. Moreover, while a computer storage medium is not a propagated signal, a computer storage medium can be a source or destination of computer program instructions encoded in an artificially-generated propagated signal. The computer storage medium can also be, or be included in, one or more separate physical components or media (e.g., solid-state memory that forms part of a device, disks, or other storage devices). For example, a component may be, but is not limited to being, a process running on a processor, an object, an executable, a thread of execution, a program, and/or a computer. By way of illustration, both an application running on a computing device and the computing device can be a component. One or more components can reside within a process and/or thread of execution and a component may be localized on one computer and/or distributed between two or more computers. In addition, these components can execute from various computer readable media having various data structures stored thereon. The components may communicate by way of local and/or remote processes such as in accordance with a signal having one or more data packets, such as data from one component interacting with another component in a local system, distributed system, and/or across a network such as the Internet with other systems by way of the signal.

Previous solutions to the communication and collaboration challenges in various sectors, including healthcare, have primarily relied on conventional methods such as emails, phone calls, and physical bulletin boards. Yet, these methods often lack real-time accessibility and are prone to delays, hindering effective information sharing and teamwork. Additionally, existing digital solutions may offer limited functionality and fail to provide a seamless integration between virtual and physical environments.

Other prior solutions have utilized IT systems that manage roles and permissions for infrastructure and software management. Yet, these systems are not designed for real-time, physical-environment interactions facilitated by AR and focus on managing roles and permissions within IT infrastructure rather than enabling continuous, persistent, real-time collaboration in a physical space. They also fail to address embedding virtual objects into physical spaces or facilitating persistent, context-aware collaboration. These prior solutions also fail to integrate virtual information with physical workflows.

Traditional methods for communication and collaboration in sectors like healthcare often involve emails, phone calls, and physical bulletin boards. Unfortunately, these methods lack real-time accessibility, leading to delays and inefficiencies in information sharing and decision-making. Further, existing digital solutions, while providing some level of interaction, do not offer a seamless integration between virtual and physical environments, nor do they ensure secure access to sensitive information through dynamic permission management.

In practice, the uniqueness of collaboration sessions used for video conferences and one-on-one communications, such as those conducted on videoconferencing applications (e.g., Zoom^{®}), can relate to their structured, content-driven nature. These collaboration sessions can often rely heavily on presented content such as slides, documents, and shared screens to serve as the basis for discussions and to stimulate conversation among participants. However, these collaborations are often limited to the duration of the meeting and can be confined to the virtual space, making it challenging to maintain continuity and context over time.

To resolve these and other issues faced in this space, the herein disclosed methods and systems extend these collaboration sessions into an ongoing, persistent state that is embedded within the real-world environment. In some aspects, the systems and methods can "breadcrumb" focal points using patient-relevant information or other contextual markers. A network of discussion points can be created that are distributed throughout physical spaces. This approach advantageously ensures that the collaboration can be continuous and seamlessly integrated into a daily workflow of professionals (e.g., including healthcare professionals). In some aspects, these virtual breadcrumbs can function as persistent anchors that facilitate real-time, context-aware interactions and discussions, to maintain a flow of information and to enhance the overall quality of care. This persistent state of collaboration transforms the traditional, episodic nature of meetings into a dynamic and ongoing exchange of insights and updates, driven by the needs and realities of the physical environment.

The herein disclosed methods and systems advantageously are capable of enhancing physical spaces with virtual objects for real-time interaction. While content in prior collaboration approaches have included sharing displays, videos, and documents, the herein disclosed methods and systems advantageously allow virtual AR objects to display data or simulate item within physical environments. Moreover, while prior approaches have defined and thus limited role for specific users during a particular collaborative meeting (e.g., a host or a presenter), the herein disclosed methods and systems advantageously allow users to have user roles be fluid based on real-time interaction(s) with AR objects. While prior approaches have included structured meetings with limited session duration, the herein disclosed methods and systems advantageously provide continuous, persistent collaboration embedded within the physical, real-world environment. The herein disclosed methods and systems also advantageously provide a mixed reality accessible via AR wearables that blend physical and virtual presence. The herein disclosed methods and systems also advantageously provide active engagement with the environment, direct manipulation of AR objects, and create new AR-objects in the real-world.

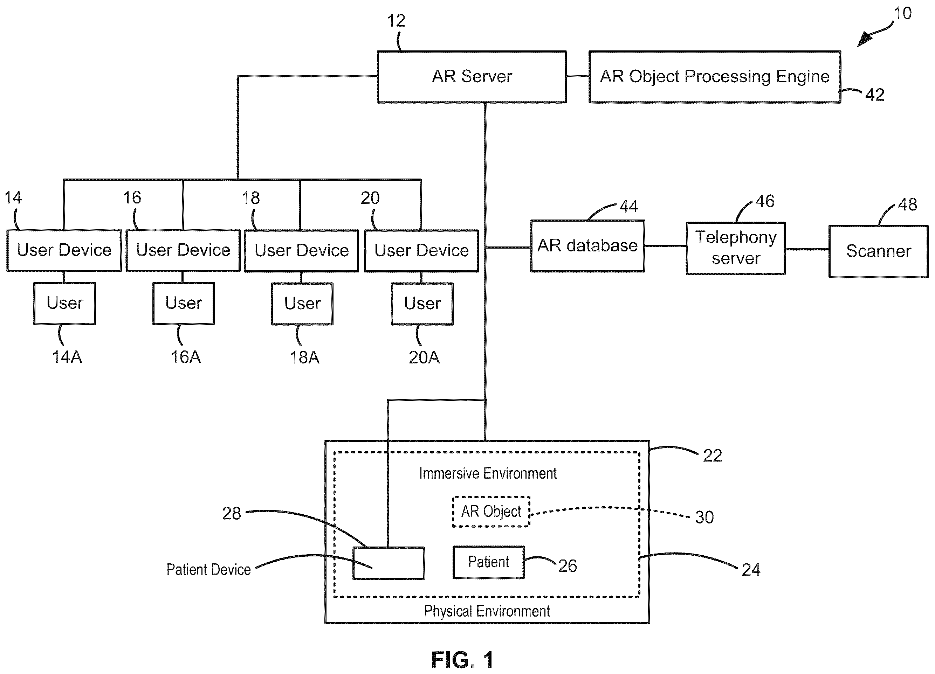

Turing to the Figures, wherein the purpose is to describe embodiments of this disclosure and not to limit the scope of the claims, FIG. 1 shows an exemplary AR system 10 for virtually positioning a virtual object into a physical environment. System 10 can include an AR server 12 that can create an immersive environment 24 superimposed on the physical environment 22. As used herein, the term "immersive environment" can refer to an artificial environment that is created with software and presentable in a way that can suspend belief and be accepted as a real, physical environment. AR server 12 can be any suitable computing device, such as a computer, processor, router, or server and can include software to provide instructions to the hardware of AR server 12 and/or may include a tangible, non-transitory memory, which includes computer program instructions that help control the operation of AR server 12. In some aspects, AR server 12 is in communication with an AR object processing engine 42. AR object processing engine 42 can generate and place an AR object 30 in the immersive environment 24 at one or more suitable locations so that a combined AR object can display combined condition data. AR object 30 can be based on a type of the condition data. AR object 30 can control to vary a format or a view of the condition data or to instruct AR object 30 to display a different condition data. In some aspects, AR object processing engine 42 can develop AR object 30 that is customized to display patient condition data.

One or more patient devices 28 can monitor condition data of a respective patient 26. Device 28 can be in communication with AR server 12 to transmit condition data to the AR server 12 and/or AR object processing engine 42. Device 28 can view or display the immersive environment 24, including the AR object 30, superimposed on physical environment 22. Device 28 can be a wearable device positioned on patient 26 or on an article of clothing of patient 26. In some aspects, device 28 can be a watch, a ring, a bracelet, an anklet, a necklace, a brooch, a tag, a glove, a hat, an item of clothing, can be included in shoes, helmets, and/or the like. Device 28 can communicate patient condition data to one or both of AR server 12 and AR object processing engine 42.

AR object 30 can be selected based on a type of the condition data and is configured to display condition data. In some aspects, AR object processing engine 42 can generate and virtually position a plurality of AR objects 30 into the physical environment 22. In some aspects, each of AR objects 30 can be at least partially based on a separate type of condition than the other of AR objects 30. AR objects 30 can display condition data for the separate type of condition to which it relates. In some aspects, device 28 can change AR object 30 to another AR object 30' (not shown). The other AR object 30' in this example can be configured to display different condition data from that displayed by the original AR object 30.

One or more user devices 14, 16, 18, and 20 (e.g., a stakeholder device) can be included in system 100 and useable by respective user 14A, 16A, 18A, and 20A. One or more user devices 14, 16, 18, and 20 can be remote to physical environment 22 as well as physically present for one or more local users. Devices 14, 16, 18, and 20 can be configured to communicate with one or more the other of the user devices. Devices 14, 16, 18, and 20 can be in communication with AR server 12. Devices 14, 16, 18, and 20 can view or display aspects related to immersive environment 24, physical environment 22, and AR object 30. Each device 14, 16, 18, and 20 can be unique to a respective user 14A, 16A, 18A, and 20A. At least one of devices 14, 16, 18, and 20, AR server 12, and/or patient device 28 can combine respective condition data from AR objects 30 in order to create a combined condition data indicative of one or more medical events. In some aspects, each unique user device 14, 16, 18, and 20 is configured to view or display immersive environment 24 and AR object 30. In some aspects, each user 14A, 16A, 18A, and 20A can have a permission rating that regulates access to AR object 30. The permission rating can in addition or separately regulate an ability to change AR object 30. Devices 14, 16, 18, and 20 can allow, based on the permission assigned to each unique user, a respective user 14A, 16A, 18A, and 20A to interact with AR object 30. In some aspects, access to AR object 30 can be limited to users 14A, 16A, 18A, and 20A with a predetermined permission rank. In some aspects, AR object(s) 30 can include one or more permissions (e.g., in a predetermined zone such as a work area) so that as respective users 14A, 16A, 18A, and 20A (e.g., workers) pass through a predetermined zone, only some, though not necessarily all, respective users 14A, 16A, 18A, and 20A can have access to AR object 30. For example, a user who is a doctor user can have a permission rank that differs from a nurse user and thus provides differing levels of access. Other such user permissions can include but are not limited to a patient's family user versus staff user. In turn, the system provides the ability to regulate access based at least partially on user permission rating(s) to ensure that sensitive information is accessible only to users 14A, 16A, 18A, and 20A with system verified access credentials.

In some aspects, permissions can be dynamically managed based on interactions with AR objects 30, such as by analyzing the interactions as well as determining whether they are in close proximity, annotating, or registering a visit (check-in), and/or the like. Attribute-based interaction(s) can ensure that only authorized users (e.g., some though potentially not all users, such as a doctor user versus a nurse user or other such staff user with a different permission rating) 14A, 16A, 18A, and 20A) can access specific AR notes, thus maintaining data security. In some aspects, if one or more users 14A, 16A, 18A, and 20A are around AR object 30 and have permissions that permit accessing and interacting with the AR object, then the one or more users 14A, 16A, 18A, and 20A with respective permissions can interact with it. In this respect, as users 14A, 16A, 18A, and 20A move through various areas, one or more AR objects 30 (e.g., dependent upon proximity to a respective work area) can become accessible for communication and collaboration.

In some aspects, the implementation of dynamic permission management logic with users ensures that sensitive information is accessible only to authorized users based on their roles and permissions. In some aspects, AR notes are generated and placed within common spaces and only visible when permissions permit (e.g., such as device 14, 16, 18, and 20 being a wearable and worn). In some aspects, only users nearby and/or with predetermined permissions are able to access and update AR objects 30 (e.g., including AR notes). In some aspects, AR objects 30 can include permissions within a predetermined area (e.g., in a work area, within a predetermined proximity, interactions with one or more AR objects 30, specific user actions such as annotating a patient visit, registering a patient visit, otherwise being involved in any manner with a patient visit, etc.) so as one or more users 14A, 16A, 18A, and 20A (e.g., such as worker users) pass through, some of those users can have access to the AR object 30 whereas other users with different permissions will not (e.g., users that differ such as a doctor versus a nurse, a patient family member versus staff, etc.). Advantageously, with AR system 10, one or more users 14A, 16A, 18A, and 20A can move through physical environment 22 and the one or more AR objects 30 can become accessible, depending one or more permissions, for communication and collaboration. AR objects 30 can operate similar to markers for ongoing, real-time discussions making patient care smoother and more efficient.

Through this collaboration, the episodic nature of meetings can be transformed into a continuous, persistent exchange of information that is seamlessly integrated into daily workflows. In some aspects, system 10 can utilize focal point "breadcrumbing" using AR objects 30, patient-relevant information, and/or other contextual markers. This is achieved through the concept of "breadcrumbing" focal points using AR objects, ensuring that collaboration is ongoing and integrated into the real-world environment. In some aspects, discussion, focal, points using AR objects 30 can be created and distributed throughout physical environment 22 so that collaboration can be ongoing, continuous, and seamlessly integrated into a daily workflow of users 14A, 16A, 18A, and 20A (e.g., including healthcare professionals). Use of virtual breadcrumbing focal points can promote real-time, context-aware interactions and discussions, to maintain information flow and overall quality of care. Advantageously, meetings that involve users 14A, 16A, 18A, and 20A (e.g., a conference session) are effectively inverted (e.g., turned inside out) so that through implementation of AR system 10, a typical videoconference session (e.g., a Zoom session) can become a reality-space medium where AR objects 30 can be overlaid turning the environment into a constantly nailed up collaboration session.

An AR database 44 may be resident on AR server 12, AR object processing engine 42, or any other suitable device. AR database 44 can be in communication with AR object 30 and can save contact information associated with one or more users who have interacted with AR object 30 and/or are affiliated with an aspect of any displayed condition data. In some aspects, during operations, one or more AR objects 30 can be imbued with contact information regarding previous participants so that respective user 14A, 16A, 18A, and 20A (e.g., a primary user of the plurality of users) can apply a communication function that associates contact information with a respective AR object 30. In some aspects, the respective user 14A, 16A, 18A, and 20A can apply on or more communication functions, including but not limited to placing a call, engaging in a message chat, and/or the like, via corresponding device14, 16, 18, and 20.

In some aspects, a telephony server 46 can be included and in communication with AR database 44 and devices 14, 16, 18, and 20. Telephony server 46 can be in communication with any number of user devices, including but not limited to devices 14, 16, 18, and provide communication protocols for delivery of digital voice communications. In some aspects, devices 14, 16, 18 can be configured to access AR database 44 and use telephony server 46 to initiate a communication with at least one of users 14A, 16A, 18A, and 20A. In some aspects, the communication can be initiated with participants users who are located within physical environment 22 and the communication can be based on respective user association with one or more AR objects 30. The communication can include, though is not limited to, placing a phone call, initiating a conference call, starting a chat session, scheduling a future meeting, and/or the like.

In some aspects, a scanner 48 can be included for assessing physical environment 22. Scanner 48 can be configured to identify one or more suitable locations for virtually positioning AR object 30 into physical environment 22. Scanner 48 can communicate the one or more suitable locations to one or both of AR server 12 and AR object processing engine 42. Scanner 48 can identify one or more suitable locations for any number of AR objects 30.

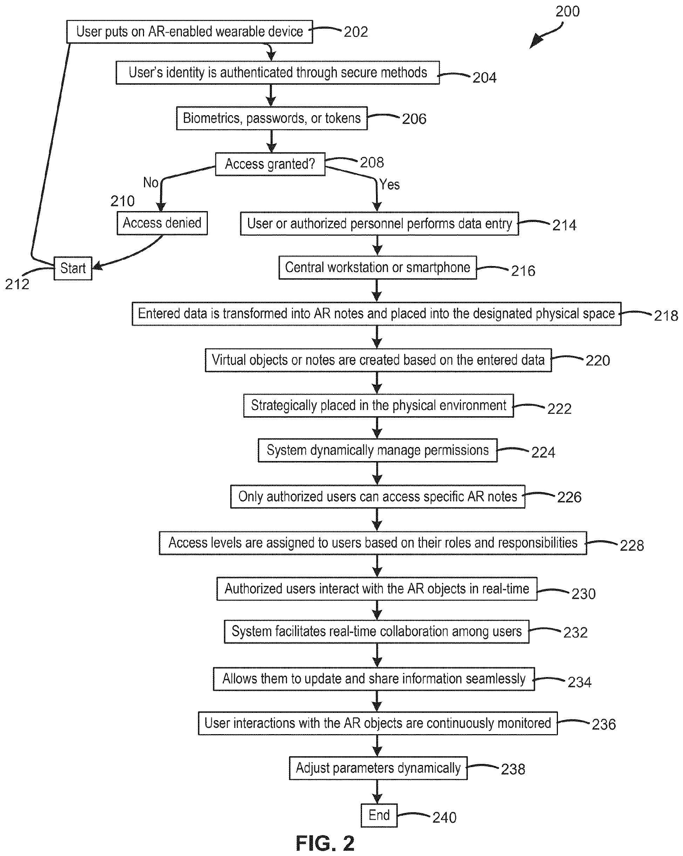

FIG. 2 is a block diagram of a method 200 of utilizing AR system (e.g., system 10) to virtually position an AR object (e.g., AR object 30) into a physical environment (e.g., physical environment 22). At step 202, a user can put on their user device, such as a wearable device (e.g., a watch, article of clothing, etc.). At step 204, the user's identity can be authenticated (e.g., by extracting user information from the wearable device and authenticated using one or more secure methods including but not limited to biometrics, passwords, tokens, as in step 206). At step 208, the system determines whether to grant access. If user authentication cannot be authenticated, then at step 210 access is denied and user authentication can be reinitiated at step 212 (e.g., returning to step 202).

If user authentication can be authenticated, then at step 214 user or authorized personnel (e.g., another user different from the user of step 202) is authorized and performs data entry. In some aspects, data entry and other aspects can be performed on a user device, such as a central workstation, personal computer, tablet, smartphone, and/or the like, as in step 216. At step 218, the entered data can be transformed by the system into AR notes and placed into the designated physical space. At step 220, virtual objects and/or AR notes are created on the entered data and at step 222, they are strategically placed in the physical environment. At step 224, the system can dynamically manage permissions (e.g., based on interactions with AR objects 30, such as by analyzing the interactions, proximity of the interactions, annotating, registered visits, etc.). At step 226, based on permissions, authorized users are able to access virtual objects and/or AR notes. In some aspects, access levels are assigned to user(s) based on roles and/or responsibilities of the respective user(s), as in step 228. In this respect, authorized user(s) can interact with the AR objects (e.g., AR object 30) in real-time, as in step 230. In step 232, the system can facilitate real-time collaboration among users so that in step 234, they can update and share information seamlessly. In step 236, the system can monitor (e.g., continuously) user interactions with the AR objects and in step 238 the parameters can be adjusted dynamically until method 200 ends at step 240. Method 200 is particularly advantageous for transforming the episodic nature of meetings into a continuous, persistent exchange of information that is seamlessly integrated into daily workflows.

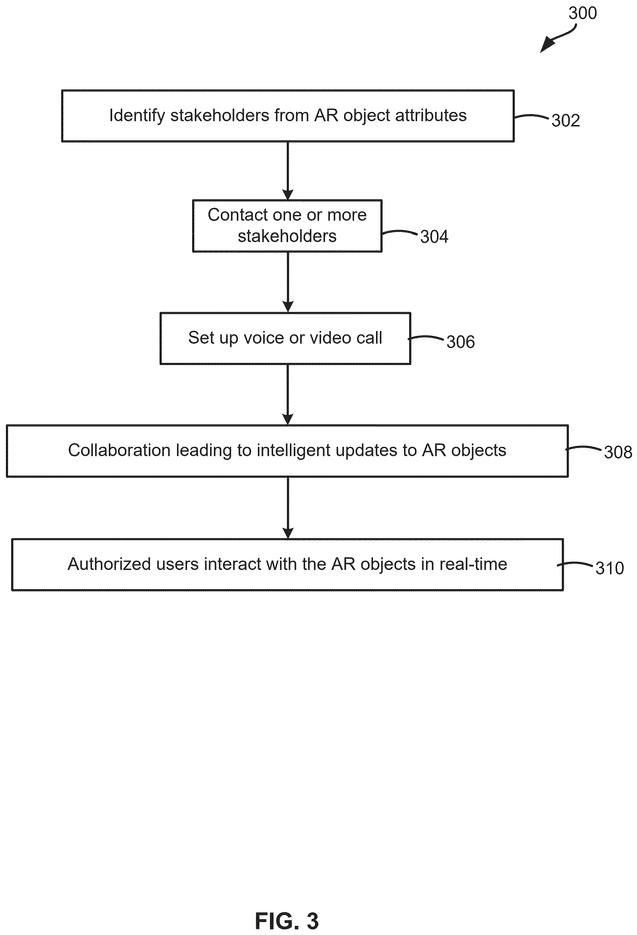

FIG. 3 is a block diagram of a method 300 for utilizing an AR system (e.g., system 10). At step 302, one or more stakeholders (e.g., users 14A, 16A, 18A, and 20A such as a clinician, doctor, nurse, cleaning staff, etc.) can be identified by the system from attributes of the AR object. At step 304, one or more of the identified stakeholders can be contacted and at step 306, a communication (e.g., a voice call, a video call, etc.) can be set up. At step 308, collaboration is performed that can lead to intelligent updates to one or more AR objects. At step 310, one or more authorized users can interact with the AR objects, including but not limited to in real-time.

According to certain embodiments, systems and methods of this disclosure can be included in user-facing front-end including software, firmware, and/or hardware to utilize an AR system integrate as discussed herein. In some embodiments, this interface to may include a mobile application ("app") or other software executable on a mobile computing device (e.g. a smart phone). It is understood that any mobile computing device of this disclosure can be configured to communicate with one or more servers. In another embodiment, the interface may include a web-based application accessible through a browser or other software, or a desktop application. The app can be configured to provide or support functionality of this disclosure.

According to certain embodiments, the one or more servers of the herein disclosed system (e.g., AR server 12) can each be connected directly or wirelessly (e.g., 3G/4G/5G, RF, a local wireless network, and/or the like). The server(s) can be operatively connected to one or more web servers across one or more networks, each server operable to permanently store and/or continuously update a database of master data. Servers of this disclosure can include back-end architecture with, or be in communication with, one or more of database server(s), whereby functionality of the system may be split between multiple servers, which may be provided by one or more discrete providers. In an example embodiment, the database server may store master data as well as logging and trace information. Software of the database server may be based on the object-relational database system PostgresSQL the database server is not so limited other approaches may be used as needed or required. This database server is not limited to only organizing and storing data and instead, it may be also used to eliminate a need of having an application server (e.g., 2nd Layer). In some embodiments, almost every functional requirement may be realized by using the database's programming language, PL/pgSQL. The database may also provide an API to the web server for data interchange based on JSON specifications. In some embodiments, the database server may also directly interact with the described functionality of respective computing devices.

In some examples, a computer system of this disclosure can be capable of implementing aspects of the present disclosure in accordance with one or more embodiments described herein, including those examples shown in FIGS. 1-3. It should be appreciated that any computer system of examples of this disclosure may be implemented within a single computing device or a computing system formed with multiple connected computing devices. The computer system of this disclosure may be configured to perform various distributed computing tasks, in which processing and/or storage resources may be distributed among the multiple devices.

In some examples, the computer system can include a processing unit ("CPU"), a system memory, and a system bus that couples the memory to the CPU. The computer system further includes a mass storage device for storing program modules. The program modules may be operable to analyze data from any herein disclosed data feeds, databases, classify user states based on the data feeds, determine responsive actions, and/or control any related operations. The program modules may include an application for performing data acquisition and/or processing functions as described herein. In some examples, the mass storage device can be connected to the CPU through a mass storage controller connected to the bus. The mass storage device and its associated computer-storage media provide non-volatile storage for the computer system. Although the description of computer-storage media contained herein refers to a mass storage device, such as a hard disk or CD-ROM drive, it should be appreciated by those skilled in the art that computer-storage media can be any available computer storage media that can be accessed by the computer system.

According to various embodiments, the computer system may operate in a networked environment using connections to other local or remote computers through a network via a network interface unit connected to the bus. The network interface unit may facilitate connection of the computing device inputs and outputs to one or more suitable networks and/or connections such as a local area network (LAN), a wide area network (WAN), the Internet, a cellular network, a radio frequency (RF) network, a Bluetooth-enabled network, a Wi-Fi enabled network, a satellite-based network, or other wired and/or wireless networks for communication with external devices and/or systems.

In some examples, the computer system may also include an input/output controller for receiving and processing input from any of a number of input devices. Input devices may include one or more of keyboards, mice, stylus, touchscreens, microphones, audio capturing devices, and image/video capturing devices. An end user may utilize the input devices to interact with a user interface, for example a graphical user interface, for managing various functions performed by the computer system. The bus may enable the processing unit to read code and/or data to/from the mass storage device or other computer-storage media.

In some examples, the computer-storage media may represent apparatus in the form of storage elements that are implemented using any suitable technology, including but not limited to semiconductors, magnetic materials, optics, or the like. The computer-storage media may represent memory components, whether characterized as RAM, ROM, flash, or other types of technology. The computer storage media may also represent secondary storage, whether implemented as hard drives or otherwise. Hard drive implementations may be characterized as solid state or may include rotating media storing magnetically-encoded information. The program modules, which include the data feed application, may include instructions that, when loaded into the processing unit and executed, cause the computer system to provide functions associated with one or more embodiments illustrated in the figures of this disclosure. The program modules may also provide various tools or techniques by which the computer system may participate within the overall systems or operating environments using the components, flows, and data structures discussed throughout this description.

In some examples, the program modules may, when loaded into the processing unit and executed, transform the processing unit and the overall computer system from a general-purpose computing system into a special-purpose computing system. The processing unit may be constructed from any number of transistors or other discrete circuit elements, which may individually or collectively assume any number of states. More specifically, the processing unit may operate as a finite-state machine, in response to executable instructions contained within the program modules. These computer-executable instructions may transform the processing unit by specifying how the processing unit transitions between states, thereby transforming the transistors or other discrete hardware elements constituting the processing unit.

Encoding the program modules may also transform the physical structure of the computer-storage media. The specific transformation of physical structure may depend on various factors, in different implementations of this description. Examples of such factors may include but are not limited to the technology used to implement the computer-storage media, whether the computer storage media are characterized as primary or secondary storage, and the like. For example, if the computer storage media are implemented as semiconductor-based memory, the program modules may transform the physical state of the semiconductor memory, when the software is encoded therein. For example, the program modules may transform the state of transistors, capacitors, or other discrete circuit elements constituting the semiconductor memory.

As another example, the computer storage media may be implemented using magnetic or optical technology. In such implementations, the program modules may transform the physical state of magnetic or optical media, when the software is encoded therein. These transformations may include altering the magnetic characteristics of particular locations within given magnetic media. These transformations may also include altering the physical features or characteristics of particular locations within given optical media, to change the optical characteristics of those locations. Other transformations of physical media are possible without departing from the scope of the present description, with the foregoing examples provided only to facilitate this discussion.

In some examples, a stakeholder user (e.g., a doctor) can be in a hospital setting and be donning a device configured according to aspects of this disclosure, such as in a wearable device (e.g., AR-enabled glasses). By using the wearable device and upon observing a virtual object linked to the patient's medical chart, the AR-enabled device can register the visit by the stakeholder user. The stakeholder user can add or modify diagnostic information related to the visit, using the AR-enabled wearable device or any other device (e.g., their smartphone), which can then be securely stored and can be visible to authorized users with corresponding rights permissions (e.g., certain staff such as a doctor or other such clinician). In some aspects, when another user (e.g., a nurse, a cleaning staff, another doctor, etc.) comes close to the same AR object, that another user can observe updated care plans and medication schedules. Advantageously, system users can have the latest info to provide the best care to beneficiaries, such as patients since interactions, like checking in or making notes, are also logged, creating a full history of who did what and when.

In some aspects, if a specialist is needed, such specialist(s) can be notified of these updates even if they are not on-site. The system can include contact info and details about their last visit to the AR object(s), making it easy to start a spontaneous communication (e.g., a voice and/or video call). In turn, all potential users can discuss treatment options and review diagnostic images together, in real time, right there in a shared virtual space. In some aspects, an action performed with a user device (e.g., a paired smartphone or an eye-head gesture to select a contact) can be used to initiate collaboration or request a physical presence. By using AR technology in such everyday tasks, the methods and systems can advantageously improve real-time interaction, maintain information in a secure form, and enhance collaborative processes.

In some examples, the systems and methods of this disclosure resolve issues with prior approaches by leveraging AR technology to create an immersive environment where virtual objects seamlessly integrate into physical spaces. The systems and methods of this disclosure also advantageously enhance real-time interaction and collaboration, providing a more intuitive and engaging user experience. In some aspects, the implementation of dynamic permission management ensures that sensitive information is accessible only to authorized users based on their roles and permissions, thereby maintaining data security. Furthermore, the sophisticated backend server can facilitate the creation, distribution, and interaction with virtual objects, offering a comprehensive and efficient solution for communication and collaboration.

In some aspects, the herein disclosed methods and systems can facilitate continuous, persistent real-time collaboration via one or more AR objects in the physical environment, enabling on-the-spot updates and interactions. Users equipped with AR-enabled devices can interact with these virtual objects to access patient-specific data, treatment plans, and other relevant information. Furthermore, the AR objects can be embedded with contact information (e.g., the author and the last known visitor), allowing for spontaneous collaboration sessions. This can include initiating chat sessions, voice calls, or video calls (e.g., spontaneously) directly from an AR interface which improves real-time communication and decision-making.

The features of the various embodiments may be stand alone or combined in any combination. Further, unless otherwise noted, various illustrated steps of a method can be performed sequentially or at the same time, and not necessarily be performed in the order illustrated. It will be recognized that changes and modifications may be made to the exemplary embodiments without departing from the scope of the present invention. These and other changes or modifications are intended to be included within the scope of the present invention, as expressed in the following claims.

The present invention has been described above with reference to a number of exemplary embodiments and examples. It should be appreciated that the particular embodiments shown and described herein are illustrative of the invention and its best mode and are not intended to limit in any way the scope of the invention as set forth in the claims. The features of the various embodiments may stand alone or be combined in any combination. Further, unless otherwise noted, various illustrated steps of a method can be performed sequentially or at the same time, and not necessarily be performed in the order illustrated. It will be recognized that changes and modifications may be made to the exemplary embodiments without departing from the scope of the present invention. These and other changes or modifications are intended to be included within the scope of the present invention, as expressed in the following claims.

## Claims

1. An augmented reality, AR, system configured to virtually position a virtual object into a physical environment, comprising:
an AR server configured to create an immersive environment superimposed on the physical environment;
a patient device configured to monitor a condition data of a patient, wherein the patient device is in communication with the AR server and is configured to transmit the condition data to the AR server;
an AR object processing engine in communication with the AR server, wherein the AR object processing engine is configured to generate and place an AR object in the immersive environment, wherein the AR object is selected based on a type of the condition data and is configured to display the condition data; and
one or more user devices in communication with the AR server, wherein at least one of the one or more user devices is configured to view or display the immersive environment, including the AR object, superimposed on the physical environment.

2. The AR system of claim 1, further comprising: a plurality of patient devices and the AR object processing engine is configured to generate and virtually position a plurality of AR objects into the physical environment, wherein each of the plurality of AR objects is based on a separate type of condition than the other of the plurality of AR objects and each of the plurality of AR objects is configured to display condition data for the separate type of condition to which it relates.

3. The AR system of claim 2, wherein either (a) the one or more user devices are, or (b) the AR server is, further configured to combine respective condition data from the plurality of the AR objects in order to create a combined condition data indicative of a medical event.

4. The AR system of claim 3, wherein the AR object processing engine is further configured to generate and place a combined AR object into the immersive environment and the combined AR object is configured to display the combined condition data.

5. The AR system of any preceding claim, wherein the patient device is a wearable device positioned on the patient or on an article of clothing of the patient.

6. The AR system of any preceding claim, wherein at least one of the one or more user devices is further configured to change the AR object to another AR object, wherein the another AR object is configured to display a different condition data from that displayed by the AR object.

7. The AR system of claim 6, further comprising: a plurality of users, wherein each of the plurality of users is associated with a unique one of the one or more user devices, and each of the plurality of users has a permission rating identified by dynamic permission management logic that regulates access to the AR object and regulates an ability to change the AR object.

8. The AR system of any preceding claim, further comprising:
an AR database in communication with the AR object, wherein the AR database saves contact information associated with one or more users who have (a) interacted with the AR object, or (b) are affiliated with an aspect of the displayed condition data; and
a telephony server in communication with the AR database and the one or more user devices;
wherein the one or more user devices are configured to access the AR database and using the telephony server initiate a communication with at least one of the one or more users, wherein the communication comprises placing a phone call, initiating a conference call, starting a chat session, or scheduling a future meeting.

9. The AR system of any preceding claim, wherein at least one of the one or more user devices is remote to the physical environment and/or further comprising: a plurality of user devices and each of the plurality of user devices is configured to communicate with each of the other of the plurality of user devices.

10. A computerized method utilizing an augmented reality, AR, system to virtually position an AR object into a physical environment, wherein the method comprises the steps of:
a scanner assessing the physical environment to identify one or more suitable locations for virtually positioning the AR object into the physical environment, wherein the scanner communicates the one or more suitable locations to one or both of an AR server and an AR object processing engine;
a patient device monitoring a condition of a patient, wherein the patient device is positioned on the patient or on clothing of the patient, and the patient device communicates patient condition data to one or both of the AR server and the AR object processing engine;
developing, by the AR object processing engine, an AR object customized to display the patient condition data;
generating, by the AR server, an immersive environment superimposed on the physical environment;
virtually positioning the AR object at one of the one or more suitable locations; and
displaying the condition data on the AR object.

11. The computerized method of claim 10, wherein the AR object processing engine generates a plurality of the AR objects and positions them as breadcrumbing focal points using a persistent marking mechanism distributed at contextual locations in the physical environment, wherein as a user device moves through networked areas of the physical environment, the AR object becomes accessible to the user device for communication and collaboration, enabling continuous real-time collaboration, only if a permission relationship is determined based on attributes of the AR object, interactions by the user device, and a permission rating of a user and/or the user device.

12. The computerized method of claim 10 or claim 11, wherein the scanner identifies one or more suitable locations for each of a plurality of different AR objects and/or wherein each of a plurality of users is assigned a unique user device, wherein each unique user device is configured to view or display the immersive environment and the AR object.

13. The computerized method of any of claims 10 to 12, further comprising:
implementing permissions for each user of the immersive environment in order to regulate access to the AR object and optionally wherein each user device further enables, based on the permission assigned to each unique user, the unique user to interact with the AR object.

14. An augmented reality, AR, apparatus that comprises (a) an AR server, (b) a patient device configured to monitor a condition data of a patient, (c) an object processing engine in communication with the AR server, and (d) a processor and a tangible, non-transitory memory configured to communicate with the processor, the non-transitory memory having stored thereon instructions which, when executed by the processor, are configured to cause the AR apparatus to execute a method including the following steps:
the AR server generating an immersive environment superimposed on a physical environment;
the patient device monitoring and transmitting condition data to one or both of the AR server and the object processing engine;
the object processing engine creating and virtually positioning an AR object in the physical environment, wherein creating the AR object is based on a type of the condition data; and
displaying the condition data on the AR object.

15. The AR apparatus of claim 14, wherein the AR object comprises controls to vary a format or a view of the condition data, or to instruct the AR object to display a different condition data and/or wherein the instructions, when executed by the processor, are further configured to permit access by users having a user device to the immersive environment and the AR object, wherein each user device displays, or enables a user to view, the immersive environment and the AR object and/or wherein the instructions, when executed by the processor, are further configured to limit access to the AR object to users using dynamic permission management logic so that that sensitive information related to or from the AR object is accessible only to users with user permissions identified based on user roles and/or attributes of the AR object.
